# EUROPEAN PATENT APPLICATION

(11) **EP 4 099 079 A1**
(43) Date of publication of application: **07.12.2022**
(21) Application number: 21747500.3
(22) Date of filing: 27.01.2021
(51) Int. Cl.: G02B 21/22, G02B 21/06

(54) **OPERATION MICROSCOPE AND OPHTHALMOLOGIC SYSTEM**

(30) Priority: 31.01.2020 JP 2020014675
(71) Applicant: TOPCON CORPORATION, Tokyo 174-8580 (JP)
(72) Inventor: OHMORI Kazuhiro, Tokyo 174-8580 (JP)
(74) Representative: Gagel, Roland
(86) International application number: PCT/JP2021/002712
(87) International publication number: WO 2021/153572

(57) **Abstract**

This operation microscope includes an objective lens, a first illumination optical system, a deflection unit, and an observation optical system. The first illumination optical system is disposed approximately coaxially with the optical axis of the objective lens, and configured to be able to irradiate an eye to be operated with first illumination light via the objective lens. The deflection unit deflects, in a direction crossing the optical axis, return light of the first illumination light incident from the eye to be operated via the objective lens. The observation optical system is configured to be able to guide the return light deflected by the defection unit to an eyepiece lens or an imaging element.

## Description

### [TECHNICAL FIELD]

The present invention relates to an operating microscope and an ophthalmic system.

### [BACKGROUND ART]

Operating microscopes are apparatuses for illuminating an eye to be operated with illumination light and observing an image formed by returning light of the illumination light using an observation optical system. For example, using a variable power lens system in the observation optical system, a magnified image of the eye to be operated can be observed. Such operating microscopes are used in ophthalmic surgery, such as cataract surgery, retinal and vitreous surgery, etc.

Techniques for the operating microscopes are disclosed in Patent Documents 1 to 4, for example. This type of operating microscope is configured so that an operator can observe the eye to be operated by peering through an eyepiece lens. Thereby, the observation optical system is arranged so that an optical path length becomes longer in a direction parallel to an optical axis of an objective lens arranged opposite the eye to be operated.

### [PRIOR ART DOCUMENTS]

### [PATENT DOCUMENTS]

[Patent Document 1] Japanese Unexamined Patent Publication No. 2013-27536
[Patent Document 2] Japanese Unexamined Patent Publication No. 2004-139002
[Patent Document 3] Japanese Unexamined Patent Publication No. 2018-198928
[Patent Document 4] Japanese Unexamined Patent Publication No. 2019-41833

### [SUMMARY OF THE INVENTION]

### [PROBLEMS TO BE SOLVED BY THE INVENTION]

In conventional operating microscopes, an optical axis of an observation optical system is arranged parallel to an optical axis of an objective lens. Thereby, in the configuration of the conventional operating microscope, the operating microscope becomes longer in a vertical direction and the observation optical system having a long optical path length is placed in front of the operator.

In case that images of the eye to be operated photographed using an operating microscope are displayed on a display device, when the operator tries to view a screen of the display device, the view of the operator is interrupted by the observation optical system (housing) placed in front of the operator, making it impossible to view the entire screen of the display device.

Alternatively, when a position of the display device is changed, the operator must assume an unreasonable posture each time the operator views the screen. Thereby, the burden on the operator is increased.

In particular, when the observation optical system is made more sophisticated by equipping it with an optical system such as a variable power lens system, the optical path length of the observation optical system becomes even longer, and the effect on the operator becomes more pronounced.

In addition, while the eye to be operated is not being observed, the observation optical system positioned in front of the operator may give the operator a feeling of oppression. Thereby, the burden on the operator may be further increased.

The present invention has been made in view of such circumstances, and an object of the present invention is to provide a new technique for reducing a burden on an operator observing an eye to be operated.

### [MEANS OF SOLVING THE PROBLEMS]

The first aspect of some embodiments is an operating microscope, including: an objective lens; a first illumination optical system arranged coaxially with an optical axis of the objective lens and configured to be capable of illuminating first illumination light onto an eye to be operated through the objective lens; a deflector configured to deflect returning light of the first illumination light in a direction intersecting the optical axis, the returning light being incident from the eye to be operated through the objective lens; and an observation optical system configured to be capable of guiding the returning light deflected by the deflector to an eyepiece lens or an imaging element.

In the second aspect of some embodiments, in the first aspect, the deflector includes a beam splitter configured to transmit the first illumination light and to reflect the returning light in the direction intersecting the optical axis.

In the third aspect of some embodiments, in the first aspect, the deflector includes: a beam splitter configured to reflect the first illumination light toward the objective lens and to transmit the returning light; and a reflective mirror arranged between the objective lens and the beam splitter, and configured to reflect the first illumination light toward the objective lens and to reflect the returning light toward the beam splitter.

The fourth aspect of some embodiments, in any one of the first aspect to the third aspect, further includes a display controller configured to display an image of the eye to be operated on a display means, based on a receiving result of the returning light obtained by the imaging element.

In the fifth aspect of some embodiments, in any one of the first aspect to the third aspect, the observation optical system includes: a left-eye observation optical system configured to be capable of guiding the returning light to a left-eye eyepiece lens or a left-eye imaging element, and a right-eye observation optical system configured to be capable of guiding the returning light to a right-eye eyepiece lens or a right-eye imaging element.

In the sixth aspect of some embodiments, in the third aspect, the observation optical system includes: a left-eye observation optical system configured to be capable of guiding the returning light to a left-eye eyepiece lens or a left-eye imaging element; and a right-eye observation optical system configured to be capable of guiding the returning light to a right-eye eyepiece lens or a right-eye imaging element, and the operating microscope further includes a stereo variator configured to be capable of being inserted into or removed from an optical path between the reflective mirror and the beam splitter.

The seventh aspect of some embodiments, in the fifth aspect or the sixth aspect, further includes a display controller configured to display an image of the eye to be operated on a display means, based on a receiving result of the returning light obtained by the left-eye imaging element and a receiving result of the returning light obtained by the right-eye imaging element.

In the eighth aspect of some embodiments, in the seventh aspect, the display controller is configured to display an image for left eye and an image for right eye on the display means, the image for left eye having been generated based on a receiving result of the returning light obtained by the left-eye imaging element, the image for right eye having been generated based on a receiving result of the returning light obtained by the right-eye imaging element.

The ninth aspect of some embodiments, in any one of the first aspect to the eighth aspect, further includes a second illumination optical system arranged eccentrically to the optical axis, and configured to be capable of illuminating the eye to be operated through the objective lens with second illumination light having a different color temperature from a color temperature of the first illumination light.

The tenth aspect of some embodiments, in the ninth aspect, further includes a first movement mechanism configured to move a position of an optical axis of the second illumination optical system relative to the optical axis of the objective lens.

In the eleventh aspect of some embodiments, in the ninth aspect or the tenth aspect, the color temperature of the first illumination light is lower than the color temperature of the second illumination light.

The twelfth aspect of some embodiments, in any one of the first aspect to the eleventh aspect, further includes a second movement mechanism configured to move a position of an optical axis of the first illumination optical system relative to the optical axis of the objective lens.

The thirteenth aspect of some embodiments is an ophthalmic system including the operating microscope of the fourth aspect, the seventh aspect, or the eighth aspect, and the display means.

It should be noted that the configurations according to a plurality of aspects described above can be combined arbitrarily.

### [EFFECTS OF THE INVENTION]

According to some embodiments of the present invention, a new technique for reducing a burden on an operator observing an eye to be operated can be provided.

### [BRIEF EXPLANATION OF THE DRAWINGS]

[FIG. 1] FIG. 1 is a schematic diagram illustrating an example of a configuration of an ophthalmic system according to a first embodiment.
[FIG. 2] FIG. 2 is a schematic diagram illustrating an example of a configuration of an optical system of the operating microscope according to the first embodiment.
[FIG. 3] FIG. 3 is a schematic diagram illustrating an example of a configuration of a control system of the operating microscope according to the first embodiment.
[FIG. 4] FIG. 4 is a schematic diagram illustrating an example of a configuration of an optical system of the operating microscope according to a second embodiment.
[FIG. 5] FIG. 5 is a schematic diagram illustrating an example of a configuration of an optical system of the operating microscope according to a third embodiment.
[FIG. 6] FIG. 6 is a schematic diagram illustrating an example of a configuration of a control system of the operating microscope according to the third embodiment.
[FIG. 7] FIG. 7 is a schematic diagram illustrating an example of a configuration of an optical system of the operating microscope according to a fourth embodiment.

### [MODES FOR CARRYING OUT THE INVENTION]

Exemplary embodiments of an operating microscope and an ophthalmic system according to the present invention will be described in detail with reference to the drawings. Any of the contents of the documents cited in the present specification and arbitrary known techniques may be applied to the embodiments below.

An ophthalmic system according to embodiments is used for observing (photographing) a magnified image of a subject's eye using an operating microscope for an operation (or a medical examination) in the field of ophthalmology. A site to be observed may be any site in an anterior segment or a posterior segment of an eye to be operated. Examples of the site to be observed in the anterior segment include a cornea, a corner angle, a vitreous body, a crystalline lens, and a ciliary body. Examples of the site to be observed in the posterior segment include a retina (fundus), a choroid, and a vitreous body. The site to be observed may also be any peripheral site of the eye such as an eyelid and an eye socket.

Hereinafter, a case where the operating microscope according to the embodiments is used for observing the fundus mainly will be described. However, the operating microscope according to the embodiments can be configured to allow observation of the anterior segment of the eye to be operated by inserting a lens into or removing a lens from between the eye to be operated and an objective lens, for example. Therefore, the configuration according to the embodiments can be applied to a configuration capable of observing the anterior segment.

The operating microscope may have a function as another ophthalmic apparatus, in addition to the function as a microscope used for magnified observation of the eye to be operated. Examples of the function as another ophthalmic apparatus include functions such as a function of performing OCT, a function of a laser treatment, a function of measuring an axial length, a function of measuring refractive power, a function of measuring a higher-order aberration. Another ophthalmic apparatus may have an arbitrary configuration capable of performing examination, measurement, or imaging of the eye to be operated using an optical system method.

### <First embodiment>

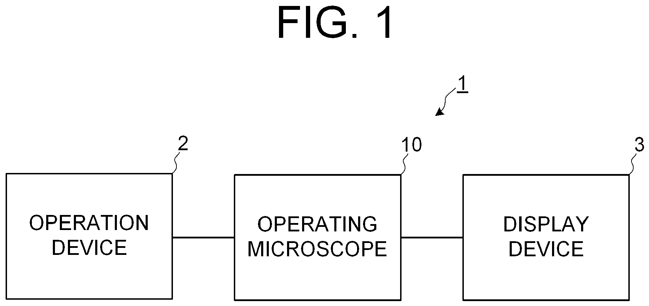

FIG. 1 shows an example of a configuration of an ophthalmic system according to the embodiments.

The ophthalmic system 1 according to the embodiments includes an operation device 2, a display device 3, and an operating microscope 10. In some embodiments, the operating microscope 10 includes at least one of the operation device 2 and the display device 3.

### (Operation device 2)

The operation device 2 includes a manipulation device or an input device. The operation device 2 includes buttons and switches (e.g., operation handle, operation knob, etc.) and manipulation devices (e.g., mouse, keyboard, etc.). In addition, the operation device 2 may include any manipulation (operation) device or any input device, such as a trackball, a control panel, a switch, a button, a dial, etc.

### (Display device 3)

The display device 3 displays an image of the eye to be operated acquired by the operating microscope 10. The display device 3 is configured to include a display device such as a flat panel display such as an LCD (Liquid Crystal Display). In addition, the display device 3 may include various types of display devices such as a touch panel.

It should be noted that the operation device 2 and the display device 3 do not need to be configured to be separate devices. For example, a device like a touch panel, which has an operation function integrated with a display function, can be used. In this case, the operation device 2 includes the touch panel and a computer program. The content for the operation device 2 is fed to the controller (not shown) as electrical signals. Moreover, operations (manipulations) and inputs of information may be performed using a graphical user interface (GUI), which is displayed on the display device 3, and the operation device 2. In some embodiments, the functions of the operation device 2 and the display device 3 are realized a touch screen.

### (Operating microscope 10)

The operating microscope 10 is used for observing a magnified image of the eye to be operated of a patient in the supine position. In some embodiments, the magnified image can be observed by displaying a captured image of the eye to be operated on the display device 3. In some embodiments, the magnified image can be observed by guiding returning light from the eye to be operated to an eyepiece lens (not shown).

In some embodiments, the operating microscope 10 includes a communication unit for sending and receiving electrical signals with the operation device 2. The operating microscope 10 is controlled according to the operation content corresponding to the electrical signals input from the operation device 2 via a wired or wireless signal path.

In some embodiments, the operating microscope 10 includes a communication unit for sending and receiving electrical signals with the display device 3. The operating microscope 10 displays images on a screen of the display device 3 according to a display control content corresponding to the electrical signals output to the display device 3 via a wired or wireless signal path.

### [Configuration of optical system]

Hereinafter, for convenience of explanation, a direction of an optical axis of an objective lens is defined as a z direction (vertical direction or perpendicular direction during operation), a horizontal direction orthogonal to the z direction is defined as an x direction (horizontal direction during operation), and a horizontal direction orthogonal to the both of the z direction and the x direction is defined as a y direction.

Further, in the following, a case where the observation optical system has an optical system for binocular observation will be described. However, the configuration according to the embodiments can also be applied to the configuration of the observation optical system having an optical system for monocular observation.

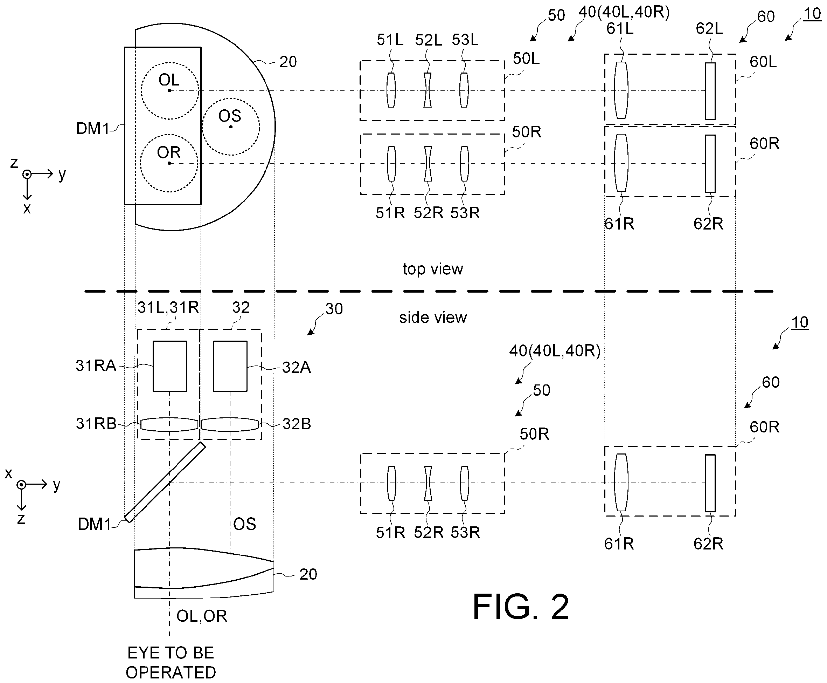

FIG. 2 shows an example of a configuration of an optical system of the operating microscope 10 according to the first embodiment. FIG. 2 shows a schematic top view of the optical system from upper side and a schematic side view of the optical system from the side correspondingly. To simplify the illustration, the illumination optical system 30, which is positioned above the objective lens 20, is omitted.

The operating microscope 10 includes an objective lens 20, a dichroic mirror DM1, an illumination optical system 30, and an observation optical system 40. The observation optical system 40 includes a zoom expander 50 and an imaging camera 60. In some embodiments, the illumination optical system 30 or the observation optical system 40 includes the dichroic mirror DM1.

### (Objective lens 20)

The objective lens 20 is positioned to face the eye to be operated on. An optical axis of objective lens 20 is assumed to extend in the z direction. In some embodiments, the objective lens 20 includes two or more lenses including a lens positioned to face the eye to be operated on.

### (Dichroic mirror DM1)

The dichroic mirror DM1 couples an optical path of the illumination optical system 30 with an optical path of the observation optical system 40. The dichroic mirror DM1 is arranged between the Illumination optical system 30 and the objective lens 20. The dichroic mirror DM1 transmits illumination light from the illumination optical system 30 to guide the illumination light to the objective lens 20, and reflects returning light of the illumination light from the objective lens 20 to guide the returning light to the imaging camera 60 of the observation optical system 40.

The dichroic mirror DM1 coaxially couples the optical path of the illumination optical system 30 with the optical path of the observation optical system 40. When the illumination optical system 30 includes a left-eye illumination optical system (31L) and a right-eye illumination optical system (31R), and the observation optical system 40 includes a left-eye observation optical system 40L and a right-eye observation optical system 40R, the dichroic mirror DM1 coaxially couples an optical path of a left-eye illumination optical system (first illumination optical system 31L) with an optical path of the left-eye observation optical system 40L, and coaxially couples an optical path of a right-eye illumination optical system (first illumination optical system 31R) with an optical path of the right-eye observation optical system 40R.

### (Illumination optical system 30)

The illumination optical system 30 is an optical system for illuminating the eye to be operated through the objective lens 20. The illumination optical system 30 can illuminate the eye to be operated with any of two or more illumination light having different color temperatures. Upon receiving instruction from a controller described below, the illumination optical system 30 illuminates the eye to be operated with illumination light having a designated color temperature.

The illumination optical system 30 according to the embodiments includes the first illumination optical systems 31L, 31R, and second illumination optical system 32.

The optical axes OL, OR of the first illumination optical systems 31L, 31R are positioned so that the optical axes are substantially coaxial with the optical axis of the objective lens 20, respectively. This allows to illumination the fundus with so-called "0-degree illumination" and to acquire a transillumination image generated by diffuse reflection of the illumination light on the fundus. In this case, the binocular observation of the transillumination image of the eye to be operated becomes possible.

An optical axis OS of the second illumination optical system 32 is eccentrically arranged relative to the optical axis of the objective lens 20. The first illumination optical systems 31L, 31R and the second illumination optical system 32 are arranged so that a displacement of the optical axis OS relative to the optical axis of the objective lens 20 is larger than a displacement of the optical axes OL, OR relative to the optical axis of the objective lens 20. This allows to illuminate the fundus or the anterior segment with so-called "angled illumination (oblique illumination)", and to binocularly observe the eye to be operated (transillumination image) while avoiding the effects of ghosting based on reflections from the cornea and other parts of the eye. Further, this also allows to observe irregularities on a predetermined site of the eye to be operated in detail.

The first illumination optical system 31L includes a light source 31LA and a condenser lens 31LB. The light source 31LA, for example, outputs illumination light having a wavelength in the visible region with a color temperature of 3000 K (Kelvin). The illumination light from the light source 31LA passes through the condenser lens 31LB, is transmitted through the dichroic mirror DM1, passes through the objective lens 20, and enters the eye to be operated.

The first illumination optical system 31R includes a light source 31RA and a condenser lens 31RB. The light source 31RA also outputs illumination light having a wavelength in the visible region with a color temperature of 3000 K, for example. The illumination light from the light source 31RA passes through the condenser lens 31RB, is transmitted through the dichroic mirror DM1, passes through the objective lens 20, and enters the eye to be operated.

The second illumination optical system 32 includes a light source 32A and a condenser lens 32B. The light source 32A, for example, outputs illumination light having a wavelength in the visible region with a color temperature of 3000 K to 6000 K. The illumination light output from the light source 32A passes through the condenser lens 32B, passes through the objective lens 20 without passing through the dichroic mirror DM1, and enters the eye to be operated.

In other words, the color temperature of the illumination light from the first illumination optical systems 31L, 31R is lower than the color temperature of the illumination light from the second illumination optical system 32. This allows to observe the eye to be operated in warm colors using the first illumination optical systems 31L, 31R, and to grasp the morphology of the eye to be operated in detail.

In some embodiments, each of the optical axes OL, OR is movable relative to the optical axis of the objective lens 20 in a direction that intersects the optical axis of the objective lens 20 (at least one of the x and y directions). In some embodiment, each of the optical axes OL, OR is independently movable. In some embodiment, the optical axes OL, OR are integrally movable. For example, the operating microscope 10 includes a movement mechanism (31d) that independently or integrally moves the first illumination optical systems 31L, 31 R, and moves the first illumination optical systems 31L, 31R independently or integrally in a direction that intersects the optical axis of the objective lens 20 using the movement mechanism. This allows to adjust the visibility of the eye to be operated. In some embodiments, the movement mechanism is controlled under the control from the controller described below.

In some embodiments, the optical axis OS is movable relative to the optical axis of the objective lens 20 in the direction that intersects the optical axis of the objective lens 20 (at least one of the x and y directions). For example, the operating microscope 10 includes a movement mechanism (32d) that moves the second illumination optical system 32, and moves the second illumination optical system 32 in the direction that intersects the optical axis of the objective lens 20 using the movement mechanism. This allows to adjust the visibility of the irregularities on a predetermined site of the eye to be operated. In some embodiments, the movement mechanism is controlled under the control from the controller described below.

As described above, the illumination optical system 30 is positioned in the transmission direction of the dichroic mirror DM1 directly above the objective lens 20, and the observation optical system 40 is positioned in the reflection direction of the dichroic mirror DM1. For example, the observation optical system 40 can be arranged so that the angle between the optical axis of the observation optical system 40 and a plane (xy-plane) orthogonal to the optical axis of the objective lens 20 is equal to or less than ±20 degrees.

Thereby, the observation optical system 40, which generally has a longer optical path length than the illumination optical system 30, is positioned so that the optical path length becomes longer in the direction substantially parallel to the xy-plane. Thus, without the observation optical system 40 being placed in front of the operator, the operator can effortlessly view the screen of the display device 3 in the front of the operator (or the situation in front of the operator). In addition, the housing placed in front of the operator no longer gives a feeling of oppression to the operator, and the burden on the operator is reduced.

### (Observation optical system 40)

The observation optical system 40 is an optical system for observing an image formed by the returning light of the illumination light incident from the eye to be operated through the objective lens 20. In the present embodiment, the observation optical system 40 images the returning light on an imaging surface of an imaging element of the imaging camera 60.

As described above, the observation optical system 40 includes a left-eye observation optical system 40L and a right-eye observation optical system 40R. The configuration of the left-eye observation optical system 40L is the same as the configuration of the right-eye observation optical system 40R. In some embodiments, the left-eye observation optical system 40L and the right-eye observation optical system 40R are configured to allow the optical arrangements to be changed independently on the left and right sides.

The zoom expander 50 includes a left-eye zoom expander 50L and a right-eye zoom expander 50R. The configuration of the left-eye zoom expander 50L is the same as the configuration of the right-eye zoom expander 50R. In some embodiments, the left-eye zoom expander 50L and the right-eye zoom expander 50R are configured to allow the optical arrangements to be changed independently on the left and right sides.

The left-eye zoom expander 50L includes a plurality of zoom lenses 51L, 52 L, 53L. Each of the plurality of zoom lenses 51L, 52L, 53L can be moved in the optical axis direction using a variable power mechanism (not shown).

The right-eye zoom expander 50R includes a plurality of zoom lenses 51R, 52 R, 53R. Each of the plurality of zoom lenses 51R, 52R, 53R can be moved in the optical axis direction using a variable power mechanism.

The variable power mechanism moves each zoom lens of the left-eye zoom expander 50L and the right-eye zoom expander 50R in the optical axis direction independently or integrally. Thereby, the magnification is changed when photographing the eye to be operated. In some embodiments, the variable power mechanism is controlled under the control from the controller described below.

### (Imaging camera 60)

The imaging camera 60 is an optical system for capturing an image formed by the returning light of the illumination light, the returning light having been guided through the observation optical system 40.

The imaging camera 60 includes a left-eye imaging camera 60L and a right-eye imaging camera 60R. The configuration of the left-eye imaging camera 60L is the same as the configuration of the right-eye imaging camera 60R. In some embodiments, the left-eye Imaging camera 60L and the right-eye Imaging camera 60R are configured to allow the optical arrangements to be changed independently on the left and right sides.

The left-eye imaging camera 60L includes an imaging lens 61L and an imaging element 62L. The imaging lens 61L images the returning light having passed through the left-eye zoom expander 50L onto the imaging surface of the imaging element 62L. The imaging element 62L is a two-dimensional area sensor. The imaging element 62L receives control from the controller described below and outputs an electrical signal (detection signal) corresponding to the light receiving result.

The right-eye imaging camera 60R includes an imaging lens 61R and an imaging element 62R. The imaging lens 61R images the returning light having passed through the right-eye zoom expander 50R onto the imaging surface of the imaging element 62R. The imaging element 62R is a two-dimensional area sensor. The imaging element 62R receives control from the controller described below and outputs an electrical signal corresponding to the light receiving result.

### [Configuration of control system]

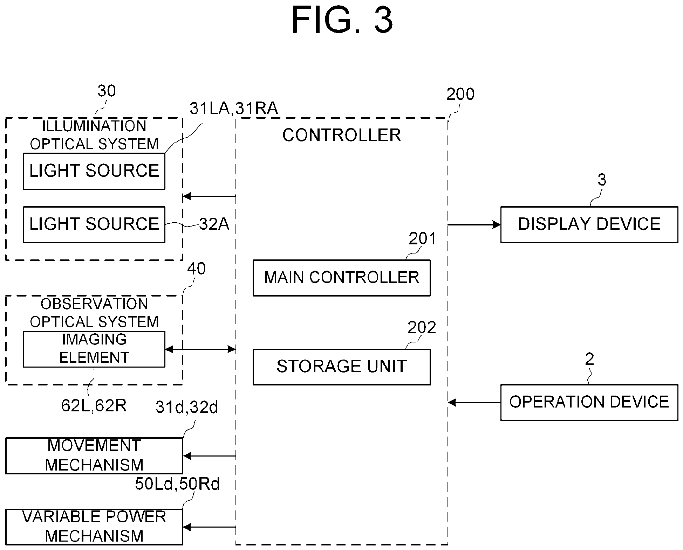

FIG. 3 shows an example of a configuration of a control system of the operating microscope 10 according to the first embodiment. In FIG. 3, like reference numerals designate like parts as in FIG. 1 or FIG. 2. The same description may not be repeated.

As shown in FIG. 3, the control system of the operating microscope 10 is configured with a controller 200 as a center. That is, the controller 200 executes control of each part of the operating microscope 10 (or ophthalmic system 1).

### (Controller 200)

The controller 200 executes various controls. The controller 200 includes a main controller 201 and a storage unit 202.

### (Main controller 201)

The main controller 201 includes a processor and controls each part of the operating microscope 10 (or ophthalmic system 1).

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the functions according to the embodiments by reading out a computer program stored in a storage circuit (storage unit 202) or a storage device and executing the computer program.

For example, the main controller 201 controls the light sources 31LA, 31RA, 32A in the illumination optical system 30, the imaging elements 62L, 62R in the observation optical system 40, the movement mechanisms 31d, 32d, the variable power mechanisms 50Ld, 50Rd, the operation device 2, the display device 3, and the like.

Examples of the control for the light source 31LA include turning on and off the light source, adjustment of the amount of light, adjustment of an aperture, and the like. Examples of the control for the light source 31RA include turning on and off the light source, adjustment of the amount of light, adjustment of an aperture, and the like. The main controller 201 performs exclusive control on the light sources 31LA, 31RA. Examples of the control for the light source 32A include turning on and off the light source, adjustment of the amount of light, adjustment of an aperture, and the like.

When the illumination optical system 30 includes a light source that can change the color temperature, the main controller 201 can control the light source to output illumination light having a desired color temperature.

Examples of the control for the imaging element 62L include adjustment of exposure, adjustment of gain, adjustment of imaging rate, and the like. Examples of the control for the imaging element 62R include adjustment of exposure, adjustment of gain, adjustment of imaging rate, and the like. In addition, the main controller 201 can control the imaging elements 62L, 62R so that the shooting timings of the imaging elements 62L, 62R coincide or the difference in shooting timings of the imaging elements 62L, 62R is within a predetermined time. Further, the main controller 201 can perform readout control of the light receiving results in the imaging elements 62L, 62R.

The movement mechanism 31d independently or integrally moves the light sources 31LA, 31RA in the direction that intersects the optical axis of the objective lens 20. The main controller 201 can control the movement mechanism 31d to independently or integrally move the optical axes OL, OR relative to the optical axis of the objective lens 20.

The movement mechanism 32d independently or integrally moves the light source 32A in the direction that intersects the optical axis of the objective lens 20. The main controller 201 can control the movement mechanism 32d to independently or integrally move the optical axis OS relative to the optical axis of the objective lens 20.

In some embodiments, the movement mechanisms 31d, 32d are configured to be cooperatively moved together. In this case, the main controller 201 can control one of the movement mechanisms 31d, 32d to move the other of the movement mechanisms 31d, 32d.

The variable power mechanism 50Ld moves at least one of the plurality of zoom lenses 51L to 53L in the left-eye zoom expander 50L in the optical axis direction. The main controller 201 can control the variable power mechanism 50Ld to move at least one of the plurality of zoom lenses 51L to 53L in the left-eye zoom expander 50L in the optical axis direction of the left-eye observation optical system 40L.

The variable power mechanism 50Rd moves at least one of the plurality of zoom lenses 51R to 53R in the right-eye zoom expander 50R in the optical axis direction. The main controller 201 can control the variable power mechanism 50Rd to move at least one of the plurality of zoom lenses 51R to 53R in the right-eye zoom expander 50R in the optical axis direction of the right-eye observation optical system 40R.

Examples of the control for the operation device 2 include an operation permission control, an operation prohibition control, and a control for receiving an operation content for operation device 2. The main controller 201 can control each part of the operating microscope 10 (or ophthalmic system 1) according to the operation contents for the operation device 2, by receiving electrical signals corresponding to the operation contents received from the operation device 2.

Examples of the control for the display device 3 include a display control of various kinds of information. The main controller 201, as a display controller, can read out the light receiving results of the imaging elements 62L, 62R to form images of the eye to be operated, and can display the formed images of the eye to be operated on the screen of the display device 3.

In addition, the main controller 201, as the display controller, can read out the light receiving results of the imaging elements 62L, 62R to form an image for left eye and an image for right eye of the eye to be operated, and can display the formed image for left eye and the formed image for right eye of the eye to be operated on the screen of the display device 3 in a manner that enables stereoscopic viewing. For example, two disparity images, one for right eye of an observer such as an operator and another for left eye of the observer, are formed from the image for left eye and the image for right eye of the eye to be operated, and the two disparity images formed are presented to the left eye and the right eye of the observer, respectively.

The operator can visually recognize the eye to be operated stereoscopically, by observing the image of the eye to be operated with the naked eye or through polarizing glasses, using known methods.

The dichroic mirror DM1 is an example of the "deflector" or the "beam splitter" according to the embodiments. The controller 200 or the main controller 201 is an example of the "display controller" according to the embodiments. The movement mechanism 32d is an example of the "first movement mechanism" according to the embodiments. The movement mechanism 31d is an example of the "second movement mechanism" according to the embodiments. The display device 3 is an example of the "display means" according to the embodiments.

As described above, according to the first embodiment, the illumination optical system 30 is positioned in the transmission direction of the dichroic mirror DM1 directly above the objective lens 20, and the observation optical system 40 is positioned in the reflection direction of the dichroic mirror DM1. Since the optical path length of the observation optical system 40 is generally longer than that of the illumination optical system 30, the space available above the eye to be operated can be increased without the observation optical system 40 placed in front of the operator. This allows the operator to view the screen of the display device 3 in front of the operator without difficulty. In addition, the housing placed in front of the operator no longer gives a feeling of oppression to the operator, and the burden on the operator is reduced.

### <Second embodiment>

The configuration of the operating microscope according to the embodiments is not limited to the configuration according to the first embodiment. In the second embodiment, a reflective mirror is placed above the objective lens 20, and the optical path of the illumination optical system 30 and the optical path of the observation optical system 40 are coupled and separated in the optical path deflected by the reflecting mirror. Hereinafter, the configuration according to the second embodiment will be described below mainly about the differences from the first embodiment.

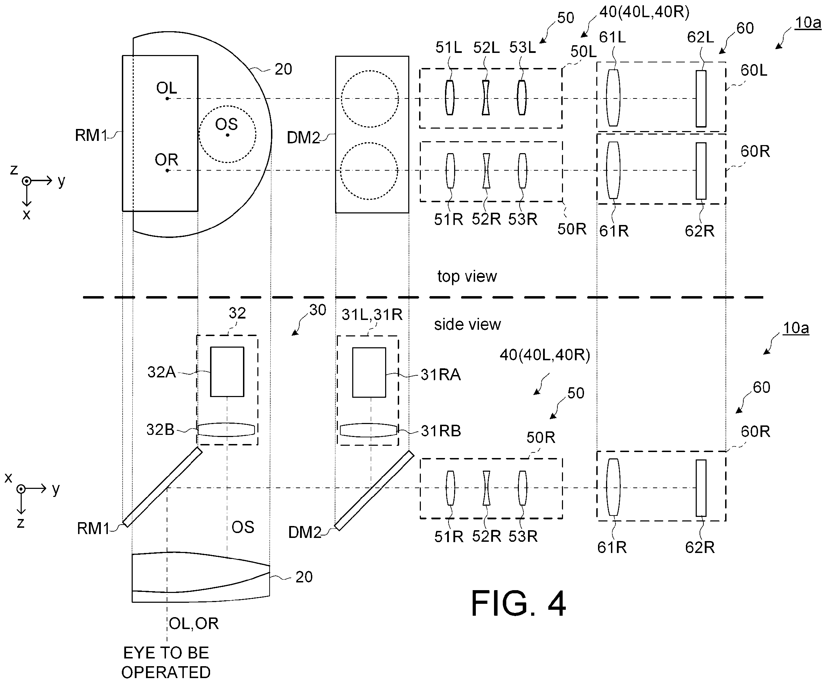

FIG. 4 shows an example of a configuration of an optical system of the operating microscope 10a according to the second embodiment. In FIG. 4, like reference numerals designate like parts as in FIG. 2, and the redundant explanation may be omitted as appropriate.

In the ophthalmic system 1 shown in FIG. 1, the operating microscope 10a according to the second embodiment can be applied instead of the operating microscope 10.

The configuration of the operating microscope 10a according to the second embodiment differs from that of the operating microscope 10 according to the first embodiment in that a reflective mirror RM1 is provided instead of the dichroic mirror DM1 and a dichroic mirror DM2 is placed in the optical path deflected by the reflective mirror RM1. The dichroic mirror DM2 couples and separates the optical paths of the illumination optical system 30 and the observation optical system 40.

The reflective mirror RM1 reflects the illumination light from the first illumination optical systems 31L, 31R toward the objective lens 20, and reflects the returning light of the illumination light from the eye to be operated toward the observation optical system 40. In the optical path deflected by the reflective mirror RM1, the dichroic mirror DM2 is placed. The dichroic mirror DM2 is arranged between the reflective mirror RM1 and the zoom expander 50 in the observation optical system 40.

The dichroic mirror DM2 couples the optical path of the illumination optical system 30 with the optical path of the observation optical system 40, in the same way as the dichroic mirror DM1. The dichroic mirror DM2 reflects the illumination light from the first illumination optical systems 31L, 31R toward the reflective mirror RM1 (objective lens 20), and transmits the returning light of the illumination light from the eye to be operated to guide the returning light to the zoom expander 50 (observation optical system 40).

The dichroic mirror DM2 coaxially couples the optical path of the illumination optical system 30 with the optical path of the observation optical system 40. When the illumination optical system 30 includes the left-eye illumination optical system (31L) and the right-eye illumination optical system (31R), and the observation optical system 40 includes the left-eye observation optical system 40L and the right-eye observation optical system 40R, the dichroic mirror DM2 coaxially couples the optical path of the left-eye illumination optical system (first illumination optical system 31L) with the optical path of the left-eye observation optical system 40L, and coaxially couples the optical path of the right-eye illumination optical system (first illumination optical system 31R) with the optical path of the right-eye observation optical system 40R.

The illumination light from the second illumination optical system 32 passes through the objective lens 20 to enter the eye to be operated without passing through the reflective mirror RM1.

A control system of the operating microscope 10a according to the second embodiment is the same as the control system of the operating microscope 10 according to the first embodiment.

In the second embodiment, the dichroic mirror DM2 is an example of the "beam splitter" according to the embodiments.

As described above, according to the second embodiment, the reflective mirror RM1 is positioned above the objective lens 20, and the coupled optical path between the optical path of the illumination optical system 30 and the optical path of the observation optical system 40 is guided to the objective lens 20. As a result, the illumination optical system 30 and the observation optical system 40 are positioned in the reflection direction of the reflective mirror RM1. Thus, without the observation optical system 40, which has a long optical path length, being placed in front of the operator, the operator can effortlessly view the screen of the display device 3. In addition, the housing placed in front of the operator no longer gives a feeling of oppression to the operator, and the burden on the operator is reduced.

### <Third embodiment>

The configuration of the operating microscope according to the embodiments is not limited to the configuration according to the embodiments described above. When the eye to be operated is a small pupil eye, it becomes difficult for the left and right illumination light to enter the eye through the pupil. Therefore, in the third embodiment, an optical axis width changing member for changing a width between the left and right optical axes is configured to be capable of being inserted into and removed from the coupled optical path between the optical path of the illumination optical system 30 and the optical path of the observation optical system 40. Hereinafter, the configuration according to the third embodiment will be described below mainly about the differences from the second embodiment.

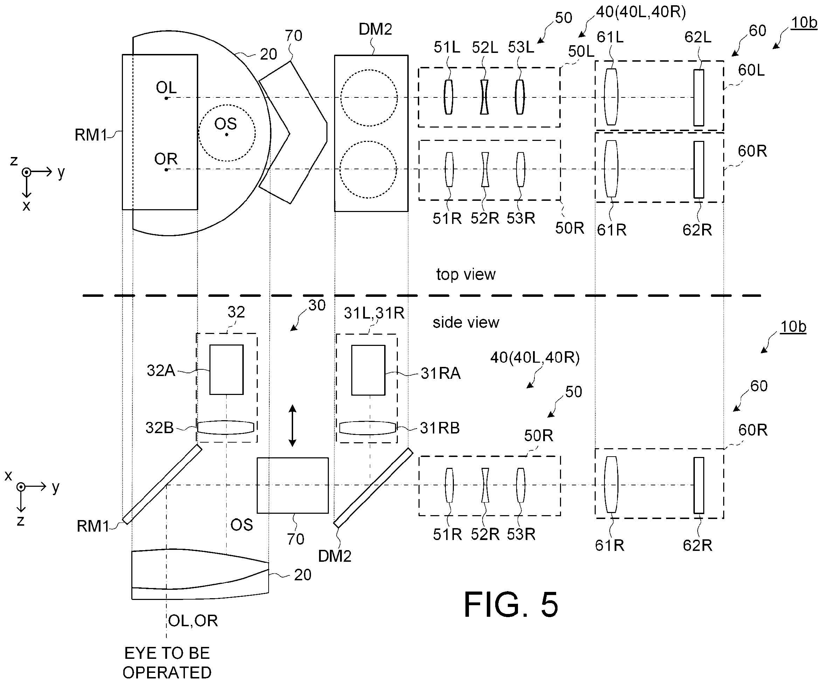

FIG. 5 shows an example of a configuration of an optical system of the operating microscope 10b according to the third embodiment. In FIG. 5, like reference numerals designate like parts as in FIG. 2 or FIG. 4. The same description may not be repeated.

In the ophthalmic system 1 shown in FIG. 1, the operating microscope 10b according to the third embodiment can be applied instead of the operating microscope 10.

The configuration of the operating microscope 10b according to the third embodiment differs from that of the operating microscope 10a according to the second embodiment in that a stereo variator 70 as the optical axis width changing member is provided so as to be capable of being inserted into and removed from the optical path.

The stereo variator 70 is, for example, as disclosed in Patent Document 2, an optical element in which a first optical member and a second optical member, each having two parallel planes, are coupled, for example. When the stereo variator 70 is placed in the optical path of the observation optical system 40, the two parallel planes of the first optical member are arranged to be inclined at a predetermined angle to the optical axis OL of the left-eye observation optical system 40L, and the two parallel planes of the second optical member are arranged to be inclined at a predetermined angle to the optical axis OR of the right-eye observation optical system 40R. Thereby, the relative positions of the optical axes OL, OR are changed, and the width between the optical axes OL, OR can be narrowed for small pupils.

The stereo variator 70 is inserted into and removed from the optical axis of the observation optical system 40, using the movement mechanism (70d) (not shown). In some embodiments, this movement mechanism is controlled under the control from a controller 200b described below.

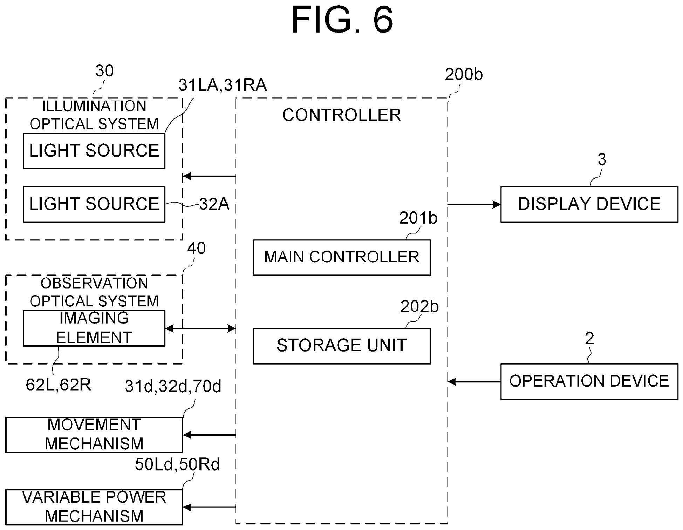

FIG. 6 shows an example of a configuration of a control system of the operating microscope 10b according to the third embodiment. In FIG. 6, like reference numerals designate like parts as in FIG. 3, and the redundant explanation may be omitted as appropriate.

As shown in FIG. 6, the control system of the operating microscope 10b is configured with the controller 200b as a center. That is, the controller 200b executes control of each part of the operating microscope 10b (or ophthalmic system 1).

The controller 200b executes various controls in the same manner as the controller 200. The controller 200b includes a main controller 201b and a storage unit 202b.

The control contents executed by controller 200b differ from those executed by controller 200 in that the control for moving the movement mechanism 70d is added.

The movement mechanism 70d positions the stereo variator 70 on the optical axis (OL, OR) of the observation optical system 40), or moves the stereo variator 70 away from the optical axis of the observation optical system 40. The main controller 201b can control the movement mechanism 70d to insert the stereo variator 70 onto the optical axis of the observation optical system 40 or to remove the stereo variator 70 from the optical axis of the observation optical system 40. In some embodiments, the main controller 201b controls the movement mechanism 70d based on the operation content for the operation device 2. In some embodiments, the main controller 201b controls the movement mechanism 70d based on an analysis result of an anterior segment image of the eye to be operated. For example, when the eye to be operated is determined to be a small pupil eye based on the analysis result of the anterior segment image, the main controller 201b controls the movement mechanism 70d to position the stereo variator 70 on the optical axis of the observation optical system 40.

As described above, according to the third embodiment, the reflective mirror RM1 is positioned above the objective lens 20, and the coupled optical path between the optical path of the illumination optical system 30 and the optical path of the observation optical system 40 is guided to the objective lens 20. As a result, the illumination optical system 30 and the observation optical system 40 are positioned in the reflection direction of the reflective mirror RM1. Then, the stereo variator 70 is inserted into or removed from the optical path of the observation optical system 40 arranged in the reflection direction of the reflective mirror RM1. Therefore, even when the eye to be operated is a small pupil eye, without the observation optical system 40, which has a long optical path length, being placed in front of the operator, the operator can view the screen of the display device 3 in front of the operator without difficulty.

### <Fourth embodiment>

The configuration of the operating microscope according to the embodiments is not limited to the configurations according to the first to the third embodiments. The fourth embodiment is configured to allow the operator or an assistant to observe the eye to be operated with the naked eye through the eyepiece lens. Hereinafter, the configuration according to the fourth embodiment will be described below mainly about the differences from the third embodiment. It should be noted that the fourth embodiment can be applied to the first embodiment or the second embodiment.

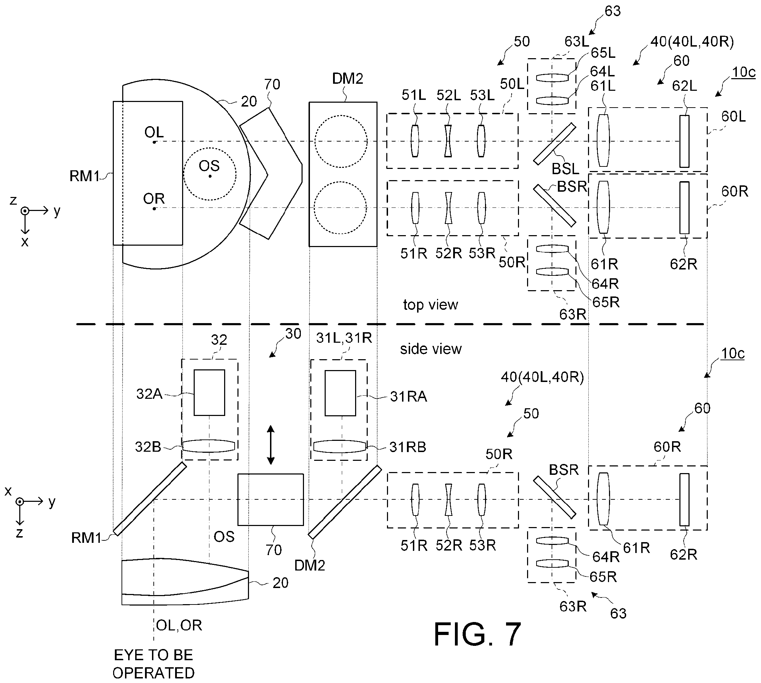

FIG. 7 shows an example of a configuration of an optical system of the operating microscope 10c according to the fourth embodiment. In FIG. 7, parts similarly configured to those in FIG. 5 are denoted by the same reference numerals, and the description thereof is omitted unless it is necessary.

In the ophthalmic system 1 shown in FIG. 1, the operating microscope 10c according to the fourth embodiment can be applied instead of the operating microscope 10.

The configuration of the operating microscope 10c according to the fourth embodiment differs from that of the operating microscope 10b according to the third embodiment in that the observation optical system 40 includes an eyepiece lens system 63.

The eyepiece lens system 63 includes a left-eye eyepiece lens system 63L and a right-eye eyepiece lens system 63R. The configuration of the left-eye eyepiece lens system 63L is the same as the configuration of the right-eye eyepiece lens system 63R. An optical path of the left-eye eyepiece lens system 63L is coaxially coupled with the optical path of the left-eye observation optical system 40L. An optical path of the right-eye eyepiece lens system 63R is coaxially coupled with the optical path of the right-eye observation optical system 40R.

A beam splitter BSL is arranged between the left-eye zoom expander 50L and the left-eye imaging camera 60L. The left-eye eyepiece lens system 63L is arranged in the reflection direction of the beam splitter BSL. The left-eye imaging camera 60L is arranged in the transmission direction of the beam splitter BSL. The beam splitter BSL coaxially couples the optical path of the left-eye eyepiece lens system 63L with the optical path of the left-eye imaging camera 60L.

The left-eye eyepiece lens system 63L includes an imaging lens 64L and an eyepiece lens 65L. The returning light of the illumination light having been guided through the optical path of the left-eye observation optical system 40L is guided to the left-eye imaging camera 60L and the left-eye eyepiece lens system 63L by the beam splitter BSL. The returning light entering the left-eye eyepiece lens system 63L passes through the imaging lens 64L and is guided to the eyepiece lens 65L.

A beam splitter BSR is arranged between the right-eye zoom expander 50R and the right-eye imaging camera 60R. The right-eye eyepiece lens system 63R is arranged in the reflection direction of the beam splitter BSR. The right-eye imaging camera 60R is arranged in the transmission direction of the beam splitter BSR. The beam splitter BSR coaxially couples the optical path of the right-eye eyepiece lens system 63R with the optical path of the right-eye imaging camera 60R.

The right-eye eyepiece lens system 63R includes an imaging lens 64R and an eyepiece lens 65R. The returning light of the illumination light having been guided through the optical path of the right-eye observation optical system 40R is guided to the right-eye imaging camera 60R and the right-eye eyepiece lens system 63R by the beam splitter BSR. The returning light entering the right-eye eyepiece lens system 63R passes through the imaging lens 64R and is guided to the eyepiece lens 65R.

A control system of the operating microscope 10c according to the fourth embodiment is the same as the control system of the operating microscope 10b according to the third embodiment.

As described above, according to the fourth embodiment, the operator or the assistant can check the eye to be operated with the naked eye and can obtain the same effect as in the third embodiment.

### [Effects]

The effects of the operating microscope and the ophthalmic system according to the embodiments will be described.

The operating microscope (10, 10a, 10b, 10c) according to the embodiments includes an objective lens (20), a first illumination optical system (30, 30L, 30R), a deflector (dichroic mirror DM1, reflective mirror RM1), and an observation optical system (40, 40L, 40R). The first illumination optical system is arranged coaxially with an optical axis of the objective lens and is configured to be capable of illuminating first illumination light onto an eye to be operated through the objective lens. The deflector is configured to deflect returning light of the first illumination light in a direction intersecting the optical axis, the returning light being incident from the eye to be operated through the objective lens. The observation optical system is configured to be capable of guiding the returning light deflected by the deflector to an eyepiece lens (65L, 65R) or an imaging element (62L, 62R).

According to such a configuration, the observation optical system is positioned in the direction that intersects the optical axis of the objective lens. Since the optical path length of the observation optical system is generally longer than that of the illumination optical system, the space available above the eye to be operated can be increased without the observation optical system placed in front of the operator. This allows the operator to grasp the situation in front of the operator without difficulty, without the view being interrupted by the observation optical system. In addition, the housing placed in front of the operator no longer gives a feeling of oppression to the operator, and the burden on the operator is reduced.

In some embodiments, the deflector includes a beam splitter (deflected surface DM1) configured to transmit the first illumination light and to reflect the returning light in the direction intersecting the optical axis described above.

According to such a configuration, the illumination optical system is positioned in the transmission direction of the beam splitter into which the returning light of the first illumination light enters through the objective lens, and the observation optical system is positioned in the reflection direction of the beam splitter. Since the optical path length of the observation optical system is generally longer than that of the illumination optical system, the space available in the optical axis direction of the eye to be operated (objective lens) can be increased without the observation optical system being placed in front of the operator. This allows the operator to grasp the situation in front of the operator without difficulty. In addition, the housing placed in front of the operator no longer gives a feeling of oppression to the operator, and the burden on the operator is reduced.

In some embodiments, the deflector includes: a beam splitter (dichroic mirror DM2) configured to reflect the first illumination light toward the objective lens and to transmit the returning light; and a reflective mirror (RM1) arranged between the objective lens and the beam splitter, and configured to reflect the first illumination light toward the objective lens and to reflect the returning light toward the beam splitter.

According to such a configuration, the reflective mirror ,into which the returning light of the first illumination light enters through the objective lens, is positioned, and the coupled optical path between the optical path of the illumination optical path and the optical path of the observation optical path is guided to the objective lens. As a result, the illumination optical system and the observation optical system are positioned in the reflection direction of the reflective mirror. Thus, without the observation optical system, which has a long optical path length, being placed in front of the operator, the operator can effortlessly grasp the situation in front of the operator. In addition, the housing placed in front of the operator no longer gives a feeling of oppression to the operator, and the burden on the operator is reduced.

Some embodiments include a display controller (controller 200, 200b, main controller 201, 201b) configured to display an image of the eye to be operated on a display means (display device 3), based on a receiving result of the returning light obtained by the imaging element.

According to such a configuration, without the observation optical system, which has a long optical path length, being placed in front of the operator, the operator can effortlessly view the image of the eye to be operated in front of the operator.

In some embodiments, the observation optical system includes: a left-eye observation optical system (40L) configured to be capable of guiding the returning light to a left-eye eyepiece lens (65L) or a left-eye imaging element (62L), and a right-eye observation optical system (40R) configured to be capable of guiding the returning light to a right-eye eyepiece lens (65R) or a right-eye imaging element (62R).

According to such a configuration, the observation optical system for binoculars is positioned in the direction that intersects the optical axis of the objective lens. Since the optical path length of the observation optical system is generally longer than that of the illumination optical system, the space available above the eye to be operated can be increased without the observation optical system being placed in front of the operator. This allows the operator to grasp the image, etc. in front of the operator without difficulty, without the view being interrupted by the observation optical system. In addition, the housing placed in front of the operator no longer gives a feeling of oppression to the operator, and the burden on the operator is reduced.

In some embodiments, the observation optical system includes: a left-eye observation optical system (40L) configured to be capable of guiding the returning light to a left-eye eyepiece lens (65L) or a left-eye imaging element (62L), and a right-eye observation optical system (40R) configured to be capable of guiding the returning light to a right-eye eyepiece lens (65R) or a right-eye imaging element (62R). The operating microscope further includes a stereo variator (70) configured to be capable of being inserted into or removed from an optical path between the reflective mirror and the beam splitter.

According to such a configuration, the observation optical system for binoculars is positioned in the direction that intersects the optical axis of the objective lens. Thereby, even when adjusting the width of the optical axes of the left and right observation optical systems for small pupils, the operator can effortlessly grasp the image, etc. without the view being interrupted by the observation optical system.

Some embodiments further include a display controller (controller 200, 200b, main controller 201, 201b) configured to display an image of the eye to be operated on a display means (display device 3), based on a receiving result of the returning light obtained by the left-eye imaging element and a receiving result of the returning light obtained by the right-eye imaging element.

According to such a configuration, without the observation optical system, which has a long optical path length, being placed in front of the operator, the operator can effortlessly view the image of the eye to be operated in front of the operator.

In some embodiments, the display controller is configured to display an image for left eye and an image for right eye on the display means, the image for left eye having been generated based on a receiving result of the returning light obtained by the left-eye imaging element, the image for right eye having been generated based on a receiving result of the returning light obtained by the right-eye imaging element.

According to such a configuration, without the observation optical system, which has a long optical path length, being placed in front of the operator, the operator can stereoscopically view the image of the eye to be operated without difficulty.

Some embodiments further include a second illumination optical system (32) arranged eccentrically to the optical axis, and configured to be capable of illuminating the eye to be operated through the objective lens with second illumination light having a different color temperature from a color temperature of the first illumination light.

according to such a configuration, the operator can observe the eye to be operated without difficulty while avoiding the effects of ghosting based on reflections from the cornea and other parts of the eye.

Some embodiments further include a first movement mechanism (movement mechanism 32d) configured to move a position of an optical axis of the second illumination optical system relative to the optical axis of the objective lens.

According to such a configuration, the incident angle of the second illumination light to the fundus can be adjusted. This allows to observe the eye to be operated without difficulty while illuminating the eye to be examined at the suitable angle for the operator and others.

In some embodiments, the color temperature of the first illumination light is lower than the color temperature of the second illumination light.

According to such a configuration, the eye to be operated can be observed in warm colors, and the morphology of the eye to be operated can be grasped in detail.

Some embodiments further include a second movement mechanism (movement mechanism 31d) configured to move a position of an optical axis of the first illumination optical system relative to the optical axis of the objective lens.

According to such a configuration, the incident angle of the first illumination light to the fundus can be adjusted. This allows to observe the eye to be operated without difficulty while illuminating the eye to be examined at the suitable angle for the operator and others.

An ophthalmic system (1) according to some embodiments includes the operating microscope described above, and the display means.

According to such a configuration, the operator can grasp the image of the eye to be operated in front the operator without difficulty, without the view being interrupted by the observation optical system. In addition, the housing placed in front of the operator no longer gives a feeling of oppression to the operator, and the burden on the operator is reduced.

The above embodiment is merely an example for implementing the present invention. Those who intend to implement the present invention may apply any modification, omission, addition, substitution, etc. within the scope of the gist of the present invention. Hereinafter, the drawings in the above embodiment will be referred to as needed.

### [Explanation of symbols]

- 1: Ophthalmic system
- 2: Operation device
- 3: Display device
- 10, 10a, 10b, 10c: Operating microscope
- 20: Objective lens
- 30: Illumination optical system
- 31L, 31R: First illumination optical system
- 32: Second illumination optical system
- 40: Observation optical system
- 40L: Left-eye observation optical system
- 40R: Right-eye observation optical system
- 50: Zoom expander
- 50L: Left-eye zoom expander
- 50R: Right-eye zoom expander
- 60: Imaging camera
- 60L: Left-eye imaging camera
- 60R: Right-eye imaging camera
- 62L, 62R: Imaging element
- 63: Eyepiece lens system
- 63L: Left-eye eyepiece lens system
- 63R: Right-eye eyepiece lens system
- 70: Stereo variator
- 200, 200b: Controller
- 201, 201b: Main controller
- 202, 202b: Storage unit
- 31d, 32d, 70d: Movement mechanism
- 50Ld, 50Rd: Variable power mechanism
- BSL, BSR: Beam splitter
- DM1, DM2: Dichroic mirror
- RM1: Reflective mirror

## Claims

1. An operating microscope, comprising:
an objective lens;
a first illumination optical system arranged coaxially with an optical axis of the objective lens and configured to be capable of illuminating first illumination light onto an eye to be operated through the objective lens;
a deflector configured to deflect returning light of the first illumination light in a direction intersecting the optical axis, the returning light being incident from the eye to be operated through the objective lens; and
an observation optical system configured to be capable of guiding the returning light deflected by the deflector to an eyepiece lens or an imaging element.

2. The operating microscope of claim 1, wherein
the deflector includes a beam splitter configured to transmit the first illumination light and to reflect the returning light in the direction intersecting the optical axis.

3. The operating microscope of claim 1, wherein
the deflector includes:
a beam splitter configured to reflect the first illumination light toward the objective lens and to transmit the returning light; and
a reflective mirror arranged between the objective lens and the beam splitter, and configured to reflect the first illumination light toward the objective lens and to reflect the returning light toward the beam splitter.

4. The operating microscope of any one of claims 1 to 3, further comprising
a display controller configured to display an image of the eye to be operated on a display means, based on a receiving result of the returning light obtained by the imaging element.

5. The operating microscope of any one of claims 1 to 3, wherein
the observation optical system includes:
a left-eye observation optical system configured to be capable of guiding the returning light to a left-eye eyepiece lens or a left-eye imaging element, and
a right-eye observation optical system configured to be capable of guiding the returning light to a right-eye eyepiece lens or a right-eye imaging element.

6. The operating microscope of claim 3, wherein
the observation optical system includes:
a left-eye observation optical system configured to be capable of guiding the returning light to a left-eye eyepiece lens or a left-eye imaging element; and
a right-eye observation optical system configured to be capable of guiding the returning light to a right-eye eyepiece lens or a right-eye imaging element, and
the operating microscope further includes a stereo variator configured to be capable of being inserted into or removed from an optical path between the reflective mirror and the beam splitter.

7. The operating microscope of claim 5 or 6, further comprising
a display controller configured to display an image of the eye to be operated on a display means, based on a receiving result of the returning light obtained by the left-eye imaging element and a receiving result of the returning light obtained by the right-eye imaging element.

8. The operating microscope of claim 7, wherein
the display controller is configured to display an image for left eye and an image for right eye on the display means, the image for left eye having been generated based on a receiving result of the returning light obtained by the left-eye imaging element, the image for right eye having been generated based on a receiving result of the returning light obtained by the right-eye imaging element.

9. The operating microscope of any one of claims 1 to 8, further comprising
a second illumination optical system arranged eccentrically to the optical axis, and configured to be capable of illuminating the eye to be operated through the objective lens with second illumination light having a different color temperature from a color temperature of the first illumination light.

10. The operating microscope of claim 9, further comprising
a first movement mechanism configured to move a position of an optical axis of the second illumination optical system relative to the optical axis of the objective lens.

11. The operating microscope of claim 9 or 10, wherein
the color temperature of the first illumination light is lower than the color temperature of the second illumination light.

12. The operating microscope of any one of claims 1 to 11, further comprising
a second movement mechanism configured to move a position of an optical axis of the first illumination optical system relative to the optical axis of the objective lens.

13. An ophthalmic system, comprising:
the operating microscope of claim 4, 7, or 8, and
the display means.
